# EUROPEAN PATENT APPLICATION

(11) **EP 4 616 853 A1**
(43) Date of publication of application: **17.09.2025**
(21) Application number: 23889175.8
(22) Date of filing: 09.11.2023
(51) Int. Cl.: A61K 31/385, A61P 27/02, A23L 33/10, C07D 339/04

(54) **LIPO-HYDROXAMIC ACID DERIVATIVE AND PHARMACEUTICAL USE THEREOF**

(30) Priority: 11.11.2022 KR 20220150854; 08.11.2023 KR 20230153791
(71) Applicant: Medicibio Co., Ltd, Yongin-si, Gyeonggi-do 17095 (KR)
(72) Inventor: KI, Min-Hyo, Yongin-si Gyeonggi-do 170958 (KR); CHOI, Mee-Hwa, Yongin-si Gyeonggi-do 16874 (KR); PARK, Jee-Young, Yongin-si Gyeonggi-do 16874 (KR); PARK, So Hyun, Yongin-si Gyeonggi-do 17011 (KR); BACK, Moon Jung, Seongnam-si Gyeonggi-do 13489 (KR); LEE, Kug Hwa, Yongin-si Gyeonggi-do 17084 (KR)
(74) Representative: Mewburn Ellis LLP
(86) International application number: PCT/KR2023/017978
(87) International publication number: WO 2024/101918

(57) **Abstract**

The present invention relates to a lipo-hydroxamic acid derivative and a pharmaceutical use thereof. The derivative exhibits excellent stability in ophthalmic formulation, and has been demonstrated to inhibit apoptosis and oxidative stress induced under corneal dystrophy-mimicking conditions in corneal endothelial cells, as well as to regulate the expression of factors associated with fibrosis and cellular degeneration, thereby being usefully applicable as a composition for the prevention, treatment, or amelioration of eye diseases.

## Description

### Technical Field

The present disclosure relates to a lipo-hydroxamic acid derivative and a pharmaceutical use thereof.

### Background Art

Lipoic acid, also known as alpha-lipoic acid or thioctic acid, is a fatty acid synthesized endogenously and localized within the mitochondria of cells. It primarily functions as a coenzyme involved in oxidation-reduction reactions, thereby exhibiting antioxidant activity within the body. In addition to its antioxidant properties, lipoic acid serves as a cofactor in the TCA cycle, a key energy production pathway. Furthermore, it plays a role in regulating glucose metabolism and acts as a chelating agent for heavy metals, thereby neutralizing and promoting the excretion of harmful metal ions from the body.

Based on such physiological effects of lipoic acid, it has been developed as a dietary supplement for health protection, and as a prescription drug for the treatment of diabetic neuropathy and diabetic neuralgia in oral dosage forms. Furthermore, its potential applicability has been proposed in the treatment of obesity, cancer, liver diseases, cardiovascular diseases, and other conditions, and active research on its bioactivity is ongoing.

Corneal dystrophy refers to a disease in which corneal opacity occurs due to genetic factors leading to malfunction of the cornea. Among the primary causes of corneal dystrophy are genetic predisposition and oxidative stress factors within the cornea. In most cases, the absence of effective pharmacological treatments renders surgery the only available option. Due to the structural characteristics of the cornea, topical treatment via eye drops may be more effective than oral administration, which faces limitations in drug delivery and distribution to the lesion site.

Although the application of lipoic acid to corneal dystrophy is conceivable, oral administration of lipoic acid is limited by its acidic structure, resulting in low bioavailability and poor distribution to the corneal tissue. Moreover, administration via mucosal routes such as the gastric or ocular mucosa may cause adverse effects including irritation or a localized sensation of heat. Therefore, research is currently underway to develop a therapeutic agent for corneal dystrophy using lipoic acid that overcomes the aforementioned side effects.

### Disclosure of the Invention

### Technical Goals

An object of the present disclosure is to provide a pharmaceutical composition for preventing or treating an ocular disease.

Another object of the present disclosure is to provide a health functional food composition for preventing or ameliorating an ocular disease.

### Technical Solutions

In order to achieve the above object, the present disclosure provides a pharmaceutical composition for preventing or treating an ocular disease, including a compound consisting of a lipo-hydroxamic acid derivative represented by the following Chemical Formula 1 or Chemical Formula 2 or a pharmaceutically acceptable salt, tautomer, stereoisomer, or optical isomer thereof as an active ingredient, wherein, in the Chemical Formula 1 and Chemical Formula 2, R₁, R₂, and R₃ may be the same or different and each independently any one of hydrogen, hydroxy, (C1-C10)alkyl, (C1-C10)alkoxy, and (C1-C10)alkyl substituted with 1 to 5 hydroxy groups.

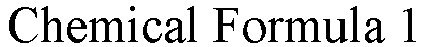

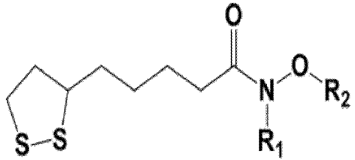

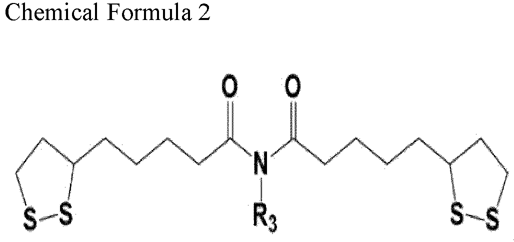

In addition, the present disclosure provides a health functional food composition for preventing or ameliorating an ocular disease, including a compound consisting of the lipo-hydroxamic acid derivative represented by Chemical Formula 1 or Chemical Formula 2 or a pharmaceutically acceptable salt, tautomer, stereoisomer, or optical isomer thereof as an active ingredient.

### Advantageous Effects

According to the present disclosure, it was determined that a lipo-hydroxamic acid derivative secures excellent stability as an eye drop composition, inhibits oxidative stress activity and apoptosis caused by corneal dystrophy in corneal endothelial cells, and regulates expression of factors related to fibrosis and cytopathic changes, and thus may be usefully utilized as a composition for preventing, treating, or ameliorating an ocular disease.

### Brief Description of Drawings

FIG. 1 is a schematic diagram showing a production process of 5-[1,2]dithiolan-3-yl-pentanoic acid (2-hydroxy-ethoxy)-amide (Compound 3; hereinafter referred to as Sample 1) in Example 1-1 below. HATU; Hexafluorophosphate Azabenzotriazole Tetramethyl Uronium, DIEA; Diisopropylethylamine, DCM; Dichloromethane.
FIG. 2 is a schematic diagram showing a production process of 5-[1,2]dithiolan-3-yl-pentanoic acid ethoxy-amide (Compound 5; hereinafter referred to as Sample 2) in Example 1-2 below. DMAP; Dimethylaminopyridine, EDCI; 1-ethyl-3-(3-dimethylamnopropyl)carbodiimide, TEA; Triethylamine, DCM; Dichrolomethane.
FIG. 3 is a schematic diagram showing a production process of (R)-5-[1,2]dithiolan-3-yl-pentanoic acid (2-hydroxy-ethoxy)-methyl-amide (Compound 8; hereinafter referred to as Sample 3) in Example 1-3 below. EtOH; Ethanol, THF; Tetrahydrofuran, HATU; Hexafluorophosphate Azabenzotriazole Tetramethyl Uronium, DIEA; Diisopropylethylamine, DCM; Dichrolomethane, Py-BH₃; (pyridin-1-ium-1-yl)boranuide.
FIG. 4 is a schematic diagram showing a production process of 5-[1,2]dithiolan-3-yl-pentanoic acid (5-[1,2]dithiolan-3-yl-pentanoyl-amide) (Compound 9; hereinafter referred to as Sample 4) in Example 1-4 below. Tol; Toluene, Py; pyridine, DMF; Dimethylformamide.
FIG. 5 is a schematic diagram showing a production process of 5-[1,2]dithiolan-3-yl-pentanoic acid (5-[1,2]dithiolan-3-yl-pentanoyl-hydroxy-amide) (Compound 10; hereinafter referred to as Sample 5) in Example 1-5 below. SOCl₂; Thionyl chloride, NH₂OPMB; O-[(4-methoxyphenyl)methyl]hydroxylamine, DIEA; Diisopropylethylamine, DCM; Dichrolomethane.
FIG. 6 shows results of analyzing an effect of lipo-hydroxamic acid derivatives (samples) prepared in Experimental Example 1 below on oxidative stress activity in corneal endothelial cells.
FIG. 7 shows results of analyzing an effect of lipo-hydroxamic acid derivatives (samples) prepared in Experimental Example 1 below on apoptosis caused by corneal dystrophy in corneal endothelial cells.
FIG. 8 shows results of analyzing a mechanism of an antioxidant activity by a lipo-hydroxamic acid derivative (sample) prepared in Experimental Example 1 below.
FIG. 9 shows results of analyzing an effect of a lipo-hydroxamic acid derivative (sample) prepared in Experimental Example 1 below on expression of factors related to fibrosis and cytopathic changes in corneal endothelial cells.
FIG. 10 shows a result of analyzing an effect of a lipo-hydroxamic acid derivative (sample) prepared in Experimental Example 1 below on apoptosis of corneal endothelial cells.
FIG. 11 shows results of analyzing an effect of a lipo-hydroxamic acid derivative (sample) prepared in Experimental Example 1 below on oxidative stress in an animal model.

### Best Mode for Carrying Out the Invention

Hereinafter, the present disclosure will be described in more detail.

The present disclosure provides a pharmaceutical composition for preventing or treating an ocular disease, including a compound including a lipo-hydroxamic acid derivative represented by the following Chemical Formula 1 or Chemical Formula 2 or a pharmaceutically acceptable salt, tautomer, stereoisomer, or optical isomer thereof as an active ingredient, wherein, in the Chemical Formula 1 and Chemical Formula 2, R₁, R₂, and R₃ may be the same or different and each independently any one of hydrogen, hydroxy, (C1-C10)alkyl, (C1-C10)alkoxy, and (C1-C10)alkyl substituted with 1 to 5 hydroxy groups.

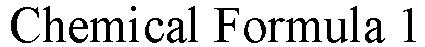

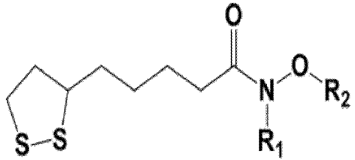

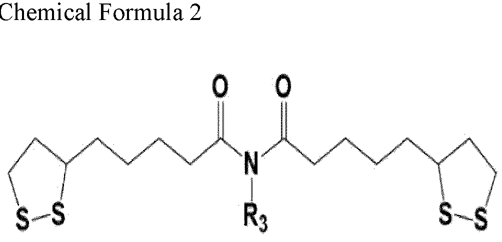

In the Chemical Formula 1, R₁ may be any one of hydrogen, hydroxy, and (C1-C5)alkyl, and preferably hydrogen or methyl.

In the Chemical Formula 1, R₂ may be any one of hydroxy, (C1-C5)alkyl, and - (C1-C5)alkyl-hydroxy, and preferably ethyl or -ethyl-hydroxy.

The compound represented by Chemical Formula 1 may be selected from the group of following compounds.
1) 5-[1,2]Dithiolan-3-yl-pentanoic acid (2-hydroxy-ethoxy)-amide;
2) 5-[1,2]Dithiolan-3-yl-pentanoic acid ethoxy-amide; and
3) 5-[1,2]Dithiolan-3-yl-pentanoic acid (2-hydroxy-ethoxy)-methyl-amide.

The 5-[1,2]dithiolan-3-yl-pentanoic acid (2-hydroxy-ethoxy)-amide may be (R)-5-[1,2]dithiolan-3-yl-pentanoic acid (2-hydroxy-ethoxy)-amide, the 5-[1,2]dithiolan-3-yl-pentanoic acid ethoxy-amide may be (R)-5-[1,2]dithiolan-3-yl-pentanoic acid ethoxy-amide, and the 5-[1,2]dithiolan-3-yl-pentanoic acid (2-hydroxy-ethoxy)-methyl-amide may be (R)-5-[1,2]dithiolan-3-yl-pentanoic acid (2-hydroxy-ethoxy)-methyl-amide.

In the Chemical Formula 2, R₃ may be any one of hydrogen, hydroxy, and (C1-C5)alkyl, and preferably hydrogen or hydroxy.

The compound represented by Chemical Formula 2 may be selected from the group of following compounds.
4) 5-[1,2]Dithiolan-3-yl-pentanoic acid (5-[1,2]dithiolan-3-yl-pentanoyl-amide); and
5) 5-[1,2]Dithiolan-3-yl-pentanoic acid (5-[1,2]dithiolan-3-yl-pentanoyl-hydroxy-amide).

The structure of the 5-[1,2]dithiolan-3-yl-pentanoic acid (2-hydroxy-ethoxy)-amide is as shown in Chemical Formula 3 below.

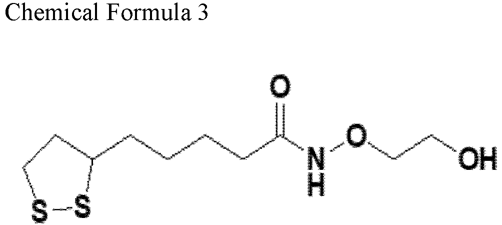

The structure of the (R)-5-[1,2]dithiolan-3-yl-pentanoic acid (2-hydroxy-ethoxy)-amide is as shown in Chemical Formula 4 below.

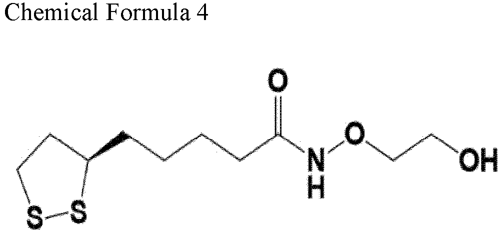

The structure of the 5-[1,2]dithiolan-3-yl-pentanoic acid ethoxy-amide is as shown in Chemical Formula 5 below.

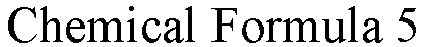

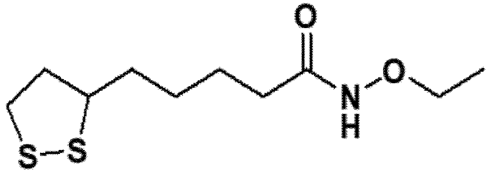

The structure of the (R)-5-[1,2]dithiolan-3-yl-pentanoic acid ethoxy-amide is as shown in Chemical Formula 6 below.

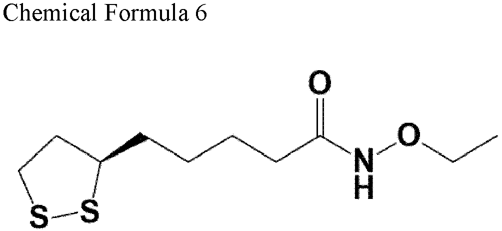

The structure of the 5-[1,2]dithiolan-3-yl-pentanoic acid (2-hydroxy-ethoxy)-methyl-amide is as shown in Chemical Formula 7 below.

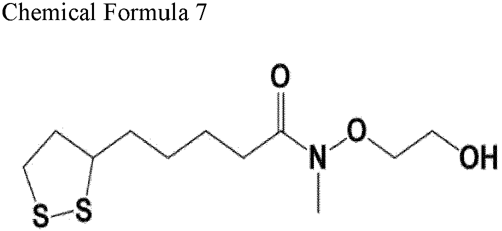

The structure of the (R)-5-[1,2]dithiolan-3-yl-pentanoic acid (2-hydroxy-ethoxy)-methyl-amide is as shown in Chemical Formula 8 below.

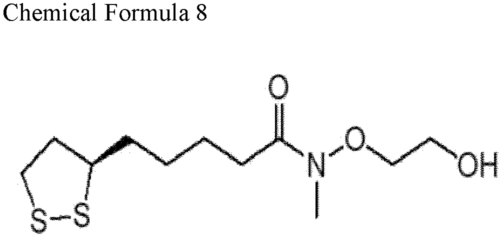

The structure of the 5-[1,2]dithiolan-3-yl-pentanoic acid (5-[1,2]dithiolan-3-yl-pentanoyl-amide) is as shown in Chemical Formula 9 below.

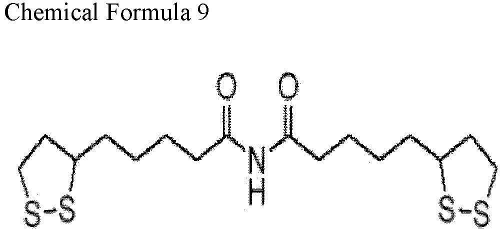

The structure of the 5-[1,2]dithiolan-3-yl-pentanoic acid (5-[1,2]dithiolan-3-yl-pentanoyl-hydroxy-amide) is as shown in Chemical Formula 10 below.

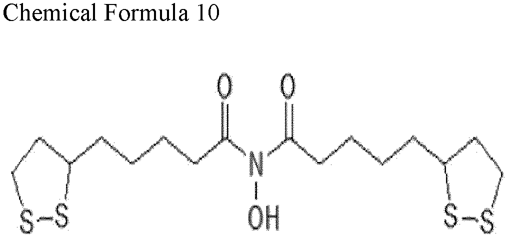

The ocular disease may be one or more selected from the group consisting of corneal dystrophy, glaucoma, cataract, keratitis, conjunctivitis, and scleritis, but is not limited thereto.

The corneal dystrophy may be one or more selected from the group consisting of Fuchs endothelial corneal dystrophy (FECD), granular corneal dystrophy 1 (GCD 1), Avellino corneal dystrophy (granular corneal dystrophy 2; GCD 2), lattice corneal dystrophy (LCD), posterior polymorphous corneal dystrophy (PPCD), and congenital hereditary endothelial dystrophy (CHED), preferably Fuchs endothelial corneal dystrophy, but is not limited thereto.

When the pharmaceutically active ingredient of the present disclosure is developed for topical ocular use, it may be used as an effective agent for the prevention and treatment of corneal dystrophy, glaucoma, cataract, keratitis, conjunctivitis, and scleritis while avoiding the side effects that occur when it takes effects systemically as an agent for oral administration.

The pharmaceutical composition of the present disclosure may be administered orally or parenterally, preferably parenterally, more preferably topically, for example, by eye drops.

The dosage of the pharmaceutical composition of the present disclosure may be adjusted depending on various factors including the type of disease, the severity of the disease, the types and contents of other ingredients contained in the pharmaceutical composition, the type of formulation, the patient's age, weight, general health, sex and diet, administration time, administration route, treatment period, and concomitant drugs.

The derivative or a pharmaceutically acceptable salt, tautomer, stereoisomer, or optical isomer thereof may be finally hydrolyzed to be converted (transformed) into lipoic acid, is neutral compared to lipoic acid to promote absorption through a biological lipid membrane, and reduces irritation to the gastrointestinal mucosa and ocular mucosa to facilitate the drug application. In addition, it may exhibit antioxidant, anti-inflammatory, and cytoprotective effects by activating the antioxidant enzyme nuclear factor erythroid 2-related factor 2 (Nrf2) and inhibiting an activity of a TGF-β signaling pathway that is overactive in corneal dystrophy.

The term "pharmaceutically acceptable salt" as used herein refers to any and all organic or inorganic addition salts of a compound that do not degrade physical properties and efficacy of the compound at a concentration in which harmless effectiveness is secured without induction of serious irritation to an organism to which the compound is administered.

The "pharmaceutically acceptable salt" refers to a salt containing a pharmaceutically acceptable cation, such as an inorganic ionic salt prepared from calcium, potassium, sodium, and magnesium; amino acid salts prepared from glycine, arginine, and lysine; and amine salts prepared from trimethylamine, triethylamine, ammonia, pyridine, and picoline. Salts containing pharmaceutically acceptable anions include acid addition salts formed by inorganic acids such as hydrochloric acid, sulfuric acid, nitric acid, phosphoric acid, hydrobromic acid (bromic acid), hydroiodic acid (iodic acid), perchloric acid, and tartaric acid; organic carbonic acids such as succinic acid, oxalic acid, tartaric acid, mandelic acid, propionic acid, citric acid, lactic acid, glycolic acid, gluconic acid, tartaric acid, formic acid, citric acid, acetic acid, trichloroacetic acid, trifluoroacetic acid, gluconic acid, benzoic acid, lactic acid, fumaric acid, maleic acid, and salicylic acid; and sulfonic acids such as methanesulfonic acid, ethanesulfonic acid, benzenesulfonic acid, p-toluenesulfonic acid, and naphthalenesulfonic acid. In the present disclosure, a preferred salt may be selected from a salt containing cations, but the present disclosure is not limited thereto. The compound according to the present disclosure may be converted into its salt by a conventional method.

The term "tautomer" as used herein refers to an interconvertible structural isomer which is an alternative form of a compound in which the positions of protons are different, such as enol-keto and imine-enamine tautomers; or a tautomeric form of a heteroaryl group having a ring atom bonded to both a ring -NH- moiety and a ring =N-moiety, such as pyrazole, imidazole, benzimidazole, triazole, and tetrazole.

The term "stereoisomer" as used herein refers to compounds having the same molecular formula and bonds with the same atomic sequence but different chiralities at one or more stereocenters. The stereoisomer includes enantiomers and diastereomers.

In addition, the present disclosure provides a health functional food composition for preventing or ameliorating an ocular disease, including a compound including a lipo-hydroxamic acid derivative represented by the following Chemical Formula 1 or Chemical Formula 2 or a pharmaceutically acceptable salt, tautomer, stereoisomer, or optical isomer thereof as an active ingredient, wherein, in the Chemical Formula 1 and Chemical Formula 2, R₁, R₂, and R₃ may be the same or different and each independently any one of hydrogen, hydroxy, (C1-C10)alkyl, (C1-C10)alkoxy, and (C1-C10)alkyl substituted with 1 to 5 hydroxy groups.

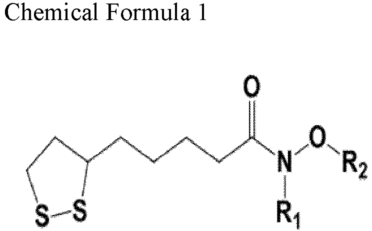

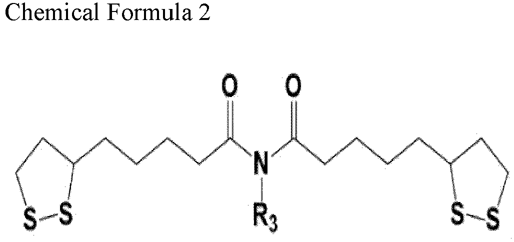

### Modes for Carrying Out the Invention

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### [Experimental Example 1] Preparation of lipo-hydroxamic acid derivatives (samples)

### 1-1. Preparation of Sample 1

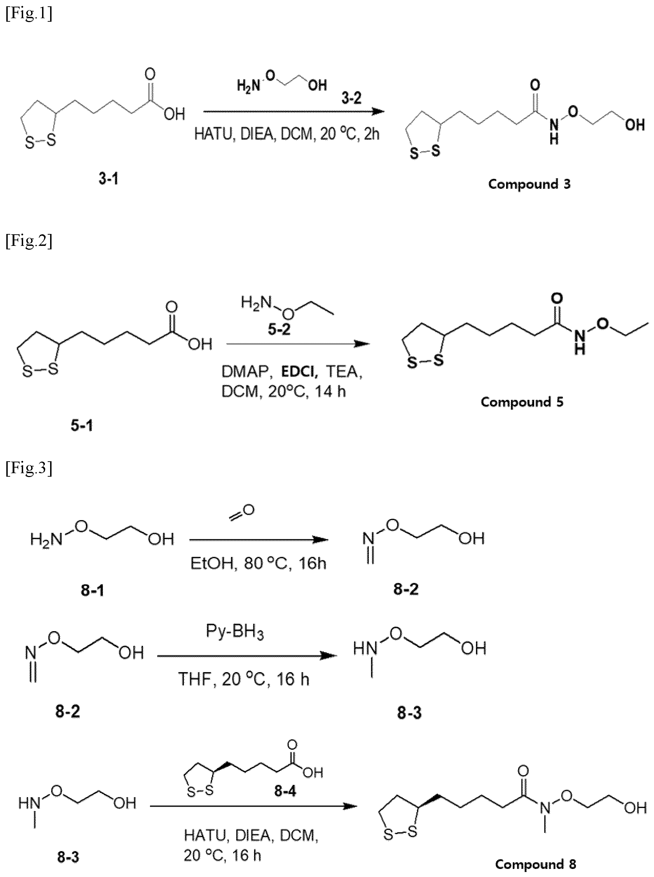

As shown in FIG. 1, Sample 1 (Compound 3) was prepared. Compound 3-1 (2 g, 9.69 mmol); Compound 3-2 (747 mg, 9.69 mmol); hexafluorophosphate azabenzotriazole tetramethyl uronium (HATU; 4.05g, 10.7mmol); and diisopropylethylamine (DIEA; 2.51 g, 19.4 mmol) were dissolved in dichloromethane (hereinafter referred to as DCM, 30 mL), which was then stirred at 20°C for 2 hours. Afterwards, the mixture was concentrated under reduced pressure, purified on silica gel (ethyl acetate (EtOAc) 100%), and purified via prep-HPLC (column C18, 5 µm, 40 × 150; mobile phase conditions: purified water-acetonitrile; 11-51% gradient, 9 minutes) to obtain Sample 1 (Compound 3) in the form of yellow solid. The NMR data of Sample 1 are as follows.

¹H NMR (400 MHz, CDCl₃) δ ppm 10.87 (s, 1 H), 4.72 (s, 1 H), 3.76 (t, J=4.8 Hz, 2 H), 3.57-3.65 (m, 1 H), 3.48-3.55 (m, 2 H), 3.07-3.22 (m, 2 H), 2.36-2.45 (m, 1 H), 1.97 (t, J=7.2 Hz, 2 H), 1.81-1.91 (m, 1 H), 1.61-1.72 (m, 1 H), 1.45-1.60 (m, 3 H), 1.29-1.39 (m, 2 H)

### 1-2. Preparation of Sample 2

As shown in FIG. 2, Sample 2 (Compound 5) was prepared. Compound 5-1 (3 g, 14.5 mmol), Compound 5-2 (1.56 mg, 16.0 mmol), dimethylaminopyridine (hereinafter referred to as DMAP; 888 mg, 7.27 mmol), and triethylamine (hereinafter referred to as TEA; 3.24 g, 32.0 mmol) were dissolved in DCM (40 mL), and 1-ethyl-3-(3-dimethylamnopropyl)carbodiimide (hereinafter referred to as EDCI; 3.62 g, 18.9 mmol) was added at 0°C, followed by stirring at 20°C for 14 hours. After that, the mixture was diluted with purified water (200 mL) and extracted with DCM (200 mL x 2), and then the organic layer was washed with saturated sodium chloride solution (hereinafter referred to as brine; 200 mL), dried with sodium sulfate (Na₂SO₄), and filtered. The filtrate was concentrated under reduced pressure and purified on silica gel (petroleum ether/ethyl acetate (hereinafter referred to as PE/EtOAc) = 1/100 to 2/3) to obtain Sample 2 (Compound 5) in the form of yellow solid. The NMR data of Sample 2 are as follows.

¹H NMR (400 MHz, DMSO-d6) δ 10.81 (s, 1H), 3.75-3.80 (m, 2H), 3.60-3.62 (m, 1H), 3.13-3.18 (m, 2H), 2.38-2.43 (m, 1H), 1.91-1.94 (m, 2H), 1.84-1.88 (m, 1H), 1.64-1.67 (m, 1H), 1.51-1.56 (m, 3H), 1.33-1.35 (m, 2H), 1.12 (t, *J =* 6.4 Hz, 3H).

### 1-3. Preparation of Sample 3

As shown in FIG. 3, Sample 3 (Compound 8) was prepared. Compound 8-1 (2 g, 26.0 mmol) and formaldehyde (935 mg, 31.1 mmol) were dissolved in ethanol (EtOH, 20 mL) and stirred at 80°C for 16 hours. Afterwards, the mixture was concentrated to obtain Compound 8-2 in the form of colorless oil. The NMR data of Compound 8-2 are as follows.

¹H NMR (400 MHz, CDCl₃) δ ppm 7.08 (d, *J =* 8.4 Hz, 1H), 6.48 (d, *J =* 8.4 Hz, 1H), 4.20-4.23 (m, 2H), 3.83-3.88 (m, 2H).

After that, Compound 8-2 (1.4 g, 15.7 mmol) and (pyridin-1-ium-1-yl)boranuide (Py-BH₃; 2.92g, 31.4mmol) were dissolved in tetrahydrofuran (THF; 15 mL), followed by stirring at 20°C for 16 hours. After that, methanol (10 mL) was added to terminate the reaction followed by stirring for 1 hour, and concentration was carried out under reduced pressure to obtain Compound 8-3 (2-(N-methylaminooxy)ethanol) in the form of colorless oil. The NMR data of Compound 8-3 are as follows.

¹H NMR (400 MHz, CDCl₃) δ ppm 4.44-4.46 (m, 2H), 3.95-3.98 (m, 2H), 2.99 (s, 3H).

After that, Compound 8-3 (4.80 g, 8.96 mmol), Compound 8-4 (5-[(3R)-dithiolan-3-yl]pentanoic acid; 1.85 g, 8.96 mmol), HATU (3.75 g, 9.85 mmol), and DIEA (2.32 g, 17.9 mmol) were dissolved in DCM (50 mL) and stirred at 20°C for 16 hours. After that, the mixture was diluted with purified water (30 mL) and extracted with DCM (30 mL x 2), and then the organic layer was washed with brine (40 mL), dried with sodium sulfate, and then filtered. The filtrate was concentrated under reduced pressure, purified on silica gel (PE/EtOAc = 4/5), purified by prep-HPLC (column C18, 5 µm, 40 × 150; mobile phase condition: purified water), and then freeze-dried to obtain Sample 3 (Compound 8) in the form of white gum. The NMR data of Sample 3 are as follows.

¹H NMR (400 MHz, DMSO-d6) δ ppm 4.82-4.88 (m, 1H), 3.86 (t, *J =* 4.4 Hz, 2H), 3.54-3.58 (m, 3H), 3.11-3.33 (m, 2H), 3.10 (s, 3H), 2.39-2.44 (m, 3H), 1.79-1.96 (m, 1H), 1.49 -1.60 (m, 4H), 1.31-1.43 (m, 2H).

### 1-4. Preparation of Sample 4

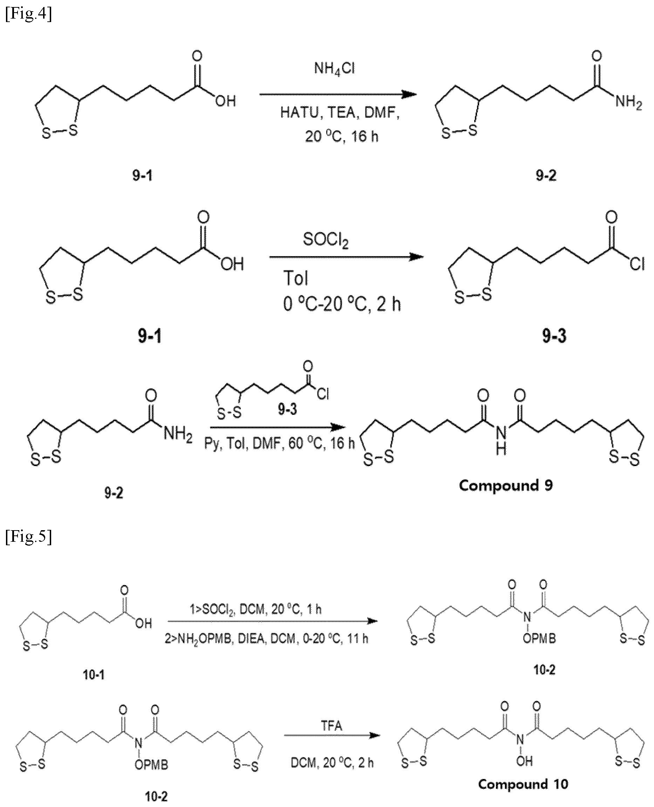

As shown in FIG. 4, Sample 4 (Compound 9) was prepared. A mixture of Compound 9-1 (10 g, 48.5 mmol), HATU (36.9 g, 96.9 mmol), TEA (9.81 g, 96.9 mmol), and ammonium chloride (NH₄Cl; 5.19 g, 96.9 mmol) was dissolved in dimethylformamide (hereinafter referred to as DMF; 300 mL) and stirred at 20°C for 16 hours. After that, the mixture was diluted with purified water (1000 mL) and extracted with DCM (500 mL × 2 times), and then the organic layer was washed with brine (500 mL), dried with sodium sulfate, and then filtered. The yellow oil obtained by concentrating the filtrate under reduced pressure was purified on silica gel (DCM/MeOH = 0 to 20/1) to obtain Compound 9-2 in the form of yellow solid. The NMR data of Compound 9-2 are as follows.

¹H NMR (400 MHz, DMSO-d6) δ 7.24 (s, 1H), 6.75 (s, 1H), 3.59-3.62 (m, 1H), 3.10-3.19 (m, 2H), 2.41-2.45 (m, 1H), 2.04 (t, *J =* 7.2 Hz, 2H), 1.86-1.87 (m, 1H), 1.61-1.72 (m, 1H), 1.48-1.52 (m, 3H), 1.33-1.36 (m, 2H).

After that, Compound 9-1 (10 g, 48.5 mmol) was dissolved in toluene (Tol; 150 mL), and thionyl chloride (SOCl₂; 14.4 g, 121 mmol) was added at 0°C, followed by stirring at 20°C for 2 hours. Afterwards, the mixture was concentrated under reduced pressure to obtain Compound 9-3 in the form of dark brown oil.

After that, Compound 9-2 (6.00 g, 29.2 mmol) was added to a mixture of pyridine (Py; 58.8 g, 743 mmol) and DMF (50 mL), and Compound 9-3 (10.0 g, 44.5 mmol) dissolved in toluene (Tol, 150 mL) was added, followed by stirring at 60°C for 16 hours. After that, the mixture was diluted with purified water (300 mL) and extracted with EtOAc (300 mL × 2), and then the organic layer was washed with 1 N hydrochloric acid solution (100 mL) and brine (300 mL), dried over sodium sulfate, and then filtered. The filtrate was concentrated under reduced pressure, purified on silica gel (PE/EtOAc = 0 to 10/3), and then purified by prep-HPLC (column C18, 5 µm, 40 × 150; mobile phase purified water-acetonitrile; 55-85% gradient, 10 min) to obtain Sample 4 (Compound 9) in the form of white solid. The NMR data of Sample 4 are as follows.

¹H NMR (400 MHz, DMSO-d6) δ 10.54 (s, 1H), 3.60-3.62 (m, 2H), 3.11-3.17 (m, 4H), 2.44-2.48 (m, 4H), 2.40-2.42 (m, 2H), 1.85-1.89 (m, 2H), 1.62-1.73 (m, 2H), 1.52-1.55 (m, 6H), 1.36-1.38 (m, 4H).

### 1-5. Preparation of Sample 5

As shown in FIG. 5, Sample 5 (Compound 10) was prepared. Compound 10-1 (20 g, 97 mmol) was added to DCM (300 mL), thionyl chloride (SOCl₂; 13.5 g, 113 mmol) was added at 0°C, which was then stirred at 20°C for 1 hour, concentrated, and then diluted with DCM (100 mL). After that, a mixture of O-[(4-methoxyphenyl)methyl]hydroxylamine (hereinafter referred to as NH₂OPMB; 6.13 g, 32.3 mmol) dissolved in DCM (100 mL) and DIEA (16.4 g, 161 mmol) was added at 20°C, which was then stirred at 20°C for 11 hours. After that, the mixture was diluted with purified water (300 mL), extracted with EtOAc (300 mL × 2), washed with brine (300 mL), and dried with sodium sulfate, followed by filtration. The filtrate was concentrated under reduced pressure and purified on silica gel (PE/EtOAc = 5/1) to obtain Compound 10-2 in the form of yellow oil. The NMR data of Compound 10-2 are as follows.

¹H NMR (400 MHz, CDCl₃) δ 7.40 (d, J=8.4 Hz, 2 H), 6.97 (d, J=8.4 Hz, 2 H), 4.88 (s, 2 H), 3.78 (s, 3 H), 3.58-3.66 (m, 2 H), 3.09-3.23 (m, 4 H), 2.69 (t, J=7.2 Hz, 4 H), 2.38-2.47 (m, 2 H), 1.83-1.92 (m, 2 H), 1.63-1.72 (m, 2 H), 1.50-1.60 (m, 6 H), 1.34-1.43 (m, 4 H)

After that, Compound 10-2 (3 g, 5.66 mmol) was dissolved in DCM (40 mL) and trifluoroacetic acid (hereinafter referred to as TFA; 15.4 g, 134 mmol), followed by stirring at 20°C for 2 hours. After that, the mixture was concentrated under reduced pressure and purified by prep-HPLC (C18 column 5 µm, 40 × 150; mobile phase conditions: purified water-acetonitrile; 47-87% gradient, 9 min) to obtain Sample 5 (Compound 10) in the form of yellow oil. The NMR data of Sample 5 are as follows.

¹H NMR (400 MHz, CDCl₃) δ 10.25 (s, 1 H), 3.57-3.66 (m, 2 H), 3.08-3.23 (m, 4 H), 2.67 (t, J=7.2 Hz, 4 H), 2.37-2.46 (m, 2 H), 1.83-1.92 (m, 2 H), 1.63-1.72 (m, 2 H), 1.50-1.61 (m, 6 H), 1.34-1.44 (m, 4 H)

### 1-6. Preparation of Samples 6-16

As shown in Table 1 below, Samples 6 to 16 were prepared. Sample 1 (Compound 3) was dissolved in propylene glycol (15 mg/mL) or dimethyl sulfoxide (10 mg/mL), purified water (normal purified water or purified water in which hydroxypropyl betadex (3 mg/mL) was dissolved) was added, and the pH was adjusted to 5.5 using a pH adjuster, followed by filtration through a 0.22 µm sterilizing filter for the preparation. Samples 9 and 14 were prepared by the above method, but mixing of Tween 80 (Polysorbate 80) was added before adding purified water. The prepared samples were stored refrigerated at 4°C.

**TABLE 1**

| | Composition (mg/mL) | | | | | | |
|---|---|---|---|---|---|---|---|
| Sample | A | B | C | D | Tween 80 | pH adjustor | Purified water |
| 6 | 1 | 15 | - | - | - | Appropriate amount | Appropriate amount |
| 7 | 1 | - | 10 | - | - | Appropriate amount | Appropriate amount |
| 8 | 1 | 15 | - | 3 | - | Appropriate amount | Appropriate amount |
| 9 | 1 | 15 | - | - | 3 | Appropriate amount | Appropriate amount |
| 10 | 1 | - | 10 | 3 | - | Appropriate amount | Appropriate amount |
| 11 | 2 | 15 | - | - | - | Appropriate amount | Appropriate amount |
| 12 | 2 | - | 10 | - | - | Appropriate amount | Appropriate amount |
| 13 | 2 | 15 | - | 3 | - | Appropriate amount | Appropriate amount |
| 14 | 2 | 15 | - | - | 3 | Appropriate amount | Appropriate amount |
| 15 | 2 | - | 10 | 3 | - | Appropriate amount | Appropriate amount |
| 16 | 2 | 15 | - | - | - | Appropriate amount | Appropriate amount |
| A: Sample 1 (Compound 3) | | | | | | | |
| B: Propylene glycol | | | | | | | |
| C: Dimethyl Sulfoxide | | | | | | | |
| D: Hydroxypropyl Betadex | | | | | | | |

### [Example 1] Analysis 1 on stability as an eye drop composition

In order to identify the stability of the lipo-hydroxamic acid derivative prepared above as an eye drop composition, the ratio (peak area, %) of the active ingredient and decomposed product of the sample (Sample 1) was determined via HPLC analysis. The HPLC analysis conditions are as shown in Table 2 below.

**TABLE 2**

| | |
|---|---|
| Column | Kromasil C18 (4.6×250 mm, 5 µm) |
| Mobile phase | 5mM potassium dihydrogen phosphate solution/methanol/acetonitrile (58/46/9) (pH adjusted to 3.0~3.1 with phosphoric acid solution) |
| UV wavelength | 215 nm |
| Column temperature | 35°C |
| Flow rate | 1.2 mL/min |

**TABLE 3**

| Peak area(%) | | | | | | | |
|---|---|---|---|---|---|---|---|
| Early stage | | Day 1 | | Day 2 | | Day 3 | |
| Sample 1 | Total amount of decomposed products | Sample 1 | Total amount of decomposed products | Sample 1 | Total amount of decomposed products | Sample 1 | Total amount of decomposed products |
| 99.72 | 0.28 | 99.36 | 0.62 | 99.20 | 0.80 | 99.36 | 0.64 |

As a result, as shown in Table 3, Sample 1 showed a slight increase in decomposed products 1 to 2 days after manufacturing, but there was no increase in decomposed products after day 3. From the above results, it was found that Sample 1 exhibited good stability in a liquid phase.

### [Example 2] Analysis of a metabolic activity in plasma

To identify the metabolic activity of the above-prepared lipo-hydroxamic acid derivative in plasma, a sample (Sample 1) was dissolved in dimethyl sulfoxide (75 mg/mL), mixed with plasma, and stored at 37°C, after which the ratio of the sample and lipoic acid was analyzed over a period of 24 hours through HPLC to determine a degree in which the sample was converted into lipoic acid. The HPLC conditions were as shown in Table 2 above, but the retention time for lipoic acid was adjusted to approximately 15 minutes.

**TABLE 4**

| Peak area (%) | | | | |
|---|---|---|---|---|
| Time (h) | 0.5 | 2 | 18 | 24 |
| Sample 1 | 97.37 | 92.65 | 17.42 | 6.00 |
| Lipoic acid | 2.63 | 7.35 | 82.58 | 94.00 |

As shown in Table 4, it was found that 90% or greater of Sample 1 was converted (metabolized) into lipoic acid 24 hours before. For general prodrugs, considering that they are hydrolyzed not only in plasma but also in water to lead to the loss of the advantages of the prodrug, it was found from the results of Examples 1 and 2 above that Sample 1 has a stable prodrug form in an eye drop composition (water) (determined in Example 1) and is quickly converted to lipoic acid only in plasma (determined in Example 2), thereby ensuring a high possibility of being utilized as a liquid formulation.

### [Example 3] Analysis of antioxidant activity

### 3-1. Cell culture

PriNeu I medium, which is a serum-free medium, was supplemented with 10% fetal bovine serum, human epidermal growth factor (EGF), human vascular endothelial growth factor (VEGF), human insulin, human transferrin, sodium selenite, hydrocortisone, heparin, β estradiol, L-glutamine, penicillin/streptomycin, and G418 (geneticin). Human corneal endothelial cells (purchased from Creative Biolabs (NY, USA)) were seeded onto culture dishes coated with FNC Coating Mix (Athena Environmental Science, MD, USA) and incubated at 37°C in a humidified 5% CO₂ atmosphere.

### 3-2. Establishment of a corneal dystrophy-like environment

To establish an intracellular environment similar to that of cells affected by corneal dystrophy (hereinafter referred to as "corneal dystrophy-like environment"), cellular signaling pathways involving reactive oxygen species (ROS) and transforming growth factor-beta (TGF-β), both known to be associated with corneal dystrophy, were activated. Human corneal endothelial cells were first cultured in standard medium for 24 hours, after which the medium was replaced with serum-free PriNeu I medium. The cells were then irradiated at an intensity of 5 J/cm² using a UV irradiation (BLX-Link, Vilber, France). Subsequently, the cells were treated with recombinant human TGF-β1 protein at a concentration of 10 ng/ml (Bon Opus, NJ, USA).

### 3-3. Analysis of in vitro antioxidant activity

To determine the effect of the above-prepared lipo-hydroxamic acid derivatives on oxidative stress activity in corneal endothelial cells, human corneal endothelial cells were cultured at 1.5×10⁴ cells per well in a 96-well plate coated with FNC Coating Mix for 18 to 24 hours. Subsequently, the cells were treated with the test samples (Samples 1, 3, and 5). Four hours after the sample treatment, oxidative stress was induced to simulate the corneal dystrophy-like environment as in Example 3-2. Sirolimus was used as a positive control group. The oxidative stress activity was determined by measuring the level of hydrogen peroxide (hereinafter referred to as "H₂O₂") in the cell culture medium using the ROS-Glo^{™} H₂O₂ Assay (Promega, WI, USA) according to the manufacturer's instructions. The luminescence intensity was measured using the GloMax^{®} Discover microplate reader (Promega) at an excitation wavelength of 485 nm and an emission wavelength of 520-530 nm.

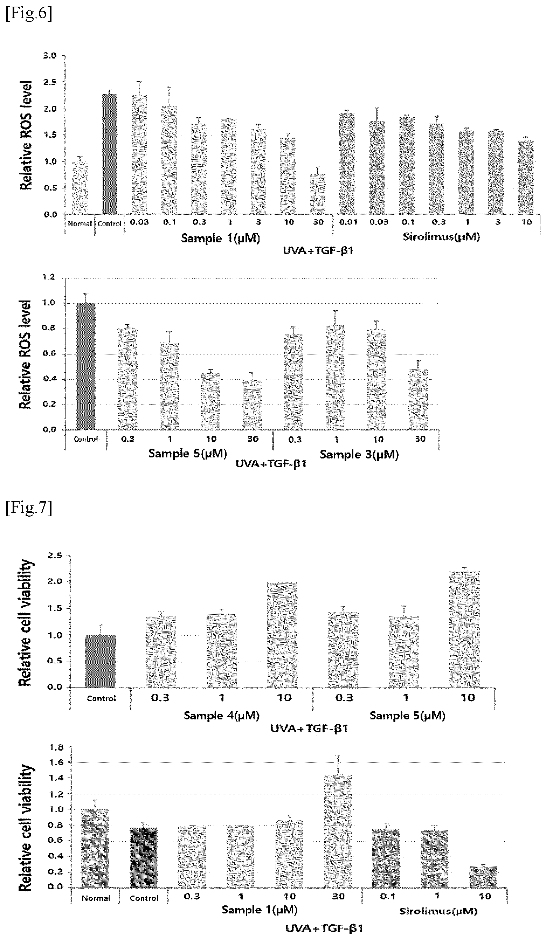

As shown in FIG. 6, oxidative stress significantly increased (approximately 2-fold increase) in the control group (corneal dystrophy-mimicking group) compared to the normal group, whereas oxidative stress significantly decreased in the sample-treated groups (Samples 1, 3, and 5) and the positive control group compared to the control group. From the above results, it was found that the lipo-hydroxamic acid derivatives (Samples 1, 3 and 5) prepared in Experimental Example 1 inhibited oxidative stress activity (caused by corneal dystrophy) in corneal endothelial cells.

### [Example 4] Analysis of anti-apoptotic effect

In order to identify the effect of the lipo-hydroxamic acid derivative prepared above on apoptosis caused by corneal dystrophy in corneal endothelial cells, human corneal endothelial cells were cultured in the same manner as in Example 3-1. Subsequently, the corneal dystrophy-like environment was induced to trigger apoptosis in the same manner as described in Example 3-2. Afterwards, the samples (Samples 1, 4, and 5) were pretreated for 24 hours, and cell viability was measured via the CellTiter-Glo 2.0 cell viability assay (Promega). The number of viable cells was determined based on intracellular ATP levels, and the luminescence intensity was measured using a GloMax Discover microplate reader (Promega).

As shown in FIG. 7, the cell viability in the control group (corneal dystrophy-mimicking group) significantly decreased (approximately 30% decrease) compared to the normal group, whereas those in the sample-treated groups showed a significant increase in viability compared to the control group. Specifically, in the Sample 1-treated group, a significant increase in cell viability was observed from a concentration of 30 µM. In the Sample 4- and Sample 5-treated groups, a significant increase was observed starting from a concentration of 0.3 µM. In contrast, the positive control group did not show a significant change in viability up to a concentration of 1 µM, and at a concentration of 10 µM, cell viability was rather decreased. These results indicate that the lipo-hydroxamic acid derivatives (Samples 1, 4, and 5) prepared in Experimental Example 1 inhibited apoptosis (caused by corneal dystrophy) in corneal endothelial cells.

### [Example 5] Analysis of a mechanism of antioxidant activity

### 5-1. Western blot

To identify a mechanism of antioxidant activity of the lipo-hydroxamic acid derivative prepared above, Western blot was performed to detect protein expression of nuclear factor erythroid-2-related factor 2 (Nrf2; NCBI Gene ID: 4780) which is an antioxidant transcription factor. First, human corneal endothelial cells were cultured at 3 × 10⁵ cells per well in a 6-well plate coated with FNC Coating Mix for 18 to 24 hours, and the samples were treated. Four hours after the sample treatment, the corneal dystrophy-like environment was induced in accordance with the method described in Example 3-2. Twenty-four hours after induction of the corneal dystrophy-like environment, the cells were washed with phosphate-buffered saline (PBS) and lysed using RIPA lysis buffer (GenDEPOT) containing protease inhibitor cocktail tablets (GenDEPOT, TX, USA). The cell lysates were centrifuged at 13,000 rpm at 4°C for 10 minutes to obtain the supernatant. Protein concentration in the supernatant was determined using a BCA kit (Thermo Fisher Scientific, MA, USA). Equal amounts of protein were mixed with 5× SDS sample buffer and boiled for 5 minutes, followed by gel electrophoresis. The separated proteins were transferred to a nitrocellulose membrane (GenDEPOT), and nonspecific antibody binding was blocked by incubating the membrane in 5% skim milk at room temperature for 1 hour. The membrane was then incubated overnight at 4°C with primary antibodies, including anti-Nrf2 (Cell Signaling Technology, MA, USA) and anti-β-actin (Cell Signaling Technology). After washing the membrane three times with TBST to remove unbound primary antibodies, it was incubated with a secondary antibody (Cell Signaling Technology). Finally, the membrane was treated with ECL reagent (Thermo Fisher Scientific) for a reaction within 1 minute, and detection was followed using a chemiluminescence imaging analysis system (Uvitec, NL, Canada).

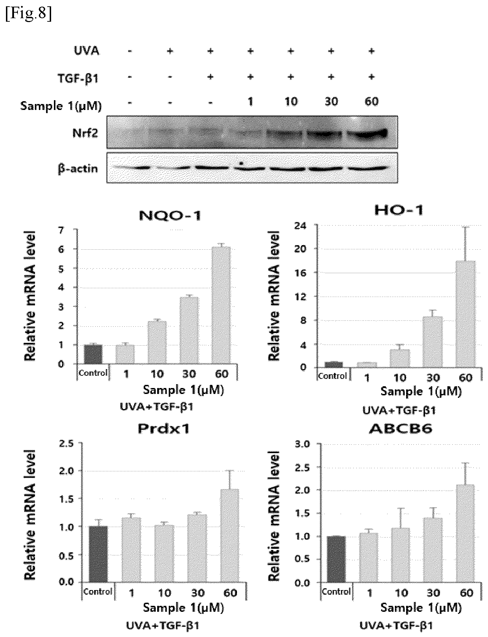

As a result, as shown in FIG. 8, it was found that Nrf2 protein expression significantly increased in the group treated with Sample 1 compared to the control.

### 5-2. Real-time PCR

To identify the antioxidant mechanism of the lipo-hydroxamic acid derivative prepared above, real-time PCR was performed to detect the mRNA expression levels of the antioxidant enzymes downstream of Nrf2. First, human corneal endothelial cells were cultured at 3 × 10⁵ cells per well in a 6-well plate coated with FNC Coating Mix for 18 to 24 hours, and the samples were treated. Four hours after the sample treatment, the corneal dystrophy-like environment was induced using the same method as described in Example 3-2. Twenty-four hours after inducing the corneal dystrophy-like environment, RNA was isolated from the cells using an RNA extraction kit (Bioneer, Daejeon), and cDNA was synthesized from 1 µg of RNA using RT PreMix (Bioneer). Subsequently, real-time PCR was performed using GoTaq mater mix (Promega) containing SYBR green solution at 95°C for 2 minutes, followed by 35 cycles at 95°C for 15 seconds and at 60°C for 1 minute. The primers used for real-time PCR are as shown in Table 5 below.

**TABLE 5**

| No. | Primer | Direction | Sequence |
|---|---|---|---|
| 1 | GAPDH | Forward | 5'-GAGTCAACGGATTTGGTCGT-3' |
| 2 | GAPDH | Reverse | 5'-GACAAGCTTCCCGTTCTCAG-3' |
| 3 | NQO-1 | Forward | 5'-GAAGCCGCAGACCTTGTGAT-3' |
| 4 | NQO-1 | Reverse | 5'-CTGCCTTCTTACTCCGGAAGG -3' |
| 5 | HO-1 | Forward | 5'-AAGACTGCGTTCCTGCTCAAC-3' |
| 6 | HO-1 | Reverse | 5'-AAAGCCCTACAGCAACTGTCG-3' |
| 7 | Prdx1 | Forward | 5'-CACTGACAAACATGGGGAAG-3' |
| 8 | Prdx1 | Reverse | 5'-TTTGCTCTTTTGGACATCAGG-3' |
| 9 | ABCB6 | Forward | 5'-GCCTCATTGTGTTCCTGTGC-3' |
| 10 | ABCB6 | Reverse | 5'-CACTTCGTAACTCTCGGCGT-3' |
| 11 | Collagen I | Forward | 5'-TCGGCGAGAGCATGACCGATGGAT-3' |
| 12 | Collagen I | Reverse | 5'-GACGCTGTAGGTGAAGCGGCTGTT-3' |
| 13 | N-cadherin | Forward | 5'-CCTGCTCTGCATCATCATCCTGC-3' |
| 14 | N-cadherin | Reverse | 5'-CTGCAGCTGGCTCAAGTCATAGTC-3' |
| 15 | Fibronectin | Forward | 5'-GCGAGAGTGCCCCTACTACA-3' |
| 16 | Fibronectin | Reverse | 5'-GTTGGTGAATCGCAGGTCA-3' |

As a result, as shown in FIG. 8, it was found that the mRNA expression of NQO-1 (NCBI Gene ID: 1728), HO-1 (NCBI Gene ID: 3162), peroxiredoxin 1 (Prdx1, NCBI Gene ID: 5052), and ABCB6 (NCBI Gene ID: 10058) significantly increased in the Sample 1-treated group.

[Example 6] Analysis of the expression of fibrosis- and cellular degeneration-related factors

### 6-1. Western blot

To identify the effect of the lipo-hydroxamic acid derivative prepared above on the expression of factors associated with fibrosis and cellular degeneration in corneal endothelial cells, Western blot was performed to detect the phosphorylation of Smad2/3 (NCBI Gene ID: 4087, 4088) protein. Western blot was performed in the same manner as in Example 5-1, but analysis was conducted using primary antibodies including anti-Smad2/3 antibody (Cell Signaling Technology, MA, USA) and anti-phospho-Smad2 (Ser465/467)/Smad3 (Ser423/425) antibody (Cell Signaling Technology) according to the manufacturer's instructions.

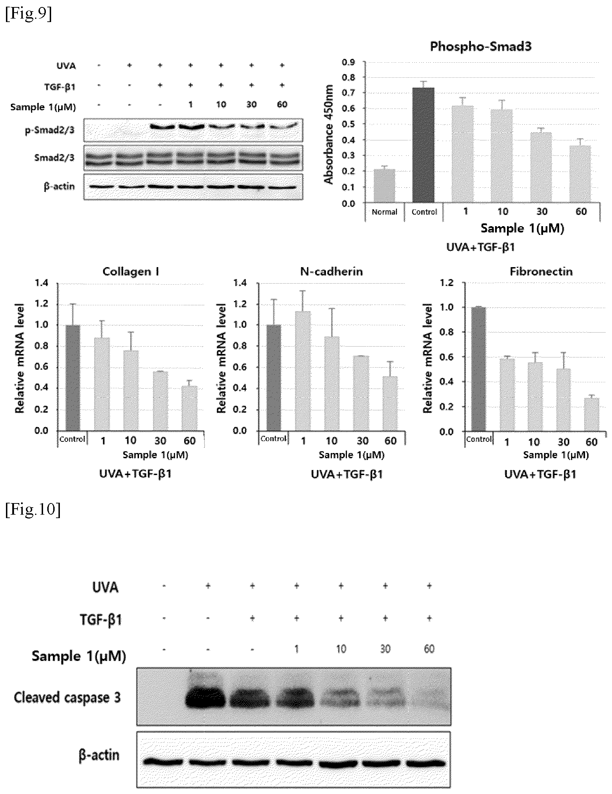

As a result, as shown in FIG. 9, the phosphorylation of Smad2/3 protein increased in control group (corneal dystrophy-mimicking group) compared to the normal group, whereas that of the protein significantly decreased in the Sample 1-treated group compared to the control group.

### 6-2. ELISA

To identify the effect of the lipo-hydroxamic acid derivative prepared above on the expression of factors associated with fibrosis- and cellular degeneration-related factors in corneal endothelial cells, ELISA was performed to observe the activity of Smad3 protein. Analysis was carried out using SMAD3 (pS423/pS425) Human InstantOne ELISA kit (Thermo Fisher Scientific) according to the manufacturer's instructions. Human corneal endothelial cells were seeded in a 96-well plate at 2×10⁴ cells per well, cultured for 24 hours, and then treated with the sample. Twenty-four hours after the sample treatment, the corneal dystrophy-like environment was induced in the same manner as in Example 3-2. Twenty-four hours after the induction, the cells were washed with PBS, lysed by adding 100 µL of lysis buffer mixture prepared by mixing the cell lysis reagent and buffer supplied in the kit. Subsequently, 50 µL of the cell lysate from each sample was added to the ELISA microplate wells. A 1: 1 mixture of capture antibody and detection antibody, as provided in the kit, was prepared to form an antibody cocktail, and 50 µL of this mixture was added to each well. The plate was sealed and incubated at room temperature on a shaker at 300 rpm for 1 hour. After incubation, the wells were washed three times using the wash buffer provided, ensuring complete removal of residual liquid. Then, 100 µL of detection reagent was added to each well and the plate was further incubated at room temperature at 300 rpm for 30 minutes. To terminate the enzymatic reaction, 100 µL of stop solution was added to each well. The plate was then read using a microplate reader (Promega) at an absorbance of 450 nm.

As a result, as shown in FIG. 9, the activity of the Smad3 protein increased in the control group (corneal dystrophy-like environmental induced group) compared to the normal group, whereas the protein activity significantly decreased in the Sample 1-treated group compared to the control group.

### 6-3. Real-time PCR

To identify the effect of the lipo-hydroxamic acid derivative prepared above on the expression of fibrosis- and cellular degeneration-related factors in corneal endothelial cells, real-time PCR was performed to detect mRNA expression levels of the fibrosis marker N-cadherin, and the cellular degeneration markers collagen I and fibronectin. The real-time PCR was performed in the same manner as in Example 5-2 above.

As a result, as shown in FIG. 9, the mRNA expression of N-cadherin (NCBI Gene ID: 1000), collagen I (NCBI Gene ID: 1277), and fibronectin (NCBI Gene ID: 2335) significantly decreased in a concentration-dependent manner in the Sample 1-treated group.

### [Example 7] Analysis of the anti-apoptotic effect in human corneal endothelial cells

To identify the effect of the lipo-hydroxamic acid derivative prepared above on apoptosis in corneal endothelial cells, Western blot was performed to detect the protein expression of cleaved caspase-3 which is a major protease involved in apoptosis. Western blot was performed in the same manner as in Example 5-1, except that in Example 7, the corneal dystrophy-like environment was induced 24 hours after sample treatment (whereas in Example 5-1, the corneal dystrophy-like environment was induced 4 hours after sample treatment). Twenty-four hours after inducing the corneal dystrophy-like environment, cells were harvested to isolate proteins. The anti-cleaved caspase-3 antibody (Asp175; Cell Signaling Technology) was used as the primary antibody, and the cleaved caspase-3 expression was detected by a chemiluminescence image analysis system.

As a result, as shown in FIG. 10, the protein expression of cleaved caspase-3 (NCBI Gene ID: 836) increased in the control group (corneal dystrophy-mimicking group) compared to the normal group, whereas the protein expression significantly decreased in a concentration-dependent manner in the Sample 1-treated group compared to the control group. From the above results, it was determined that the lipo-hydroxamic acid derivative (Sample 1) prepared in Experimental Example 1 inhibits apoptosis caused by corneal dystrophy in human corneal endothelial cells.

### [Example 8] Analysis of antioxidant activity in vivo

To determine the effect of the sample on oxidative stress, animal experiments were conducted using Sprague-Dawley rats (purchased from Bio Link). To induce oxidative stress in the cornea of an animal model, the anterior chamber of the right eye was punctured using a syringe equipped with a 30G needle, and then the needle was inserted into the corneal limbus to inject 0.02% benzalkonium (BAK), a known oxidative stress inducer. The ocular surface was subsequently rinsed with sterile saline. To determine whether oxidative stress was induced, the H₂O₂ level, an indicator of oxidative stress, was measured in the anterior aqueous humor and corneal tissue using the Amplex Red Hydrogen Peroxide/Peroxidase assay kit (Invitrogen, MA, USA) according to the manufacturer's instructions. Afterwards, the animal model received 10 µL drops (single dose) of the sample (Sample 1) at a dose of 0.1% or 0.3% for 24 hours, including pretreatment, three times a day for 3 days, and then the H₂0₂ level in the anterior aqueous humor was measured. Additionally, proteins were extracted from the harvested corneal tissue, and Western blot was performed in the same manner as in Example 5-1 to examine the expression of the Nrf2 protein. Specifically, the experimental groups were set up as follows.
1) Normal group (PBS): PBS (total 90 µL) administration
2) Control group (Vehicle): 0.02% benzalkonium administration
3) 0.1% Sample 1 administered group: 0.02% benzalkonium administration + 0.1% Sample 1 (total 90 µL) administration
4) 0.3% Sample 1 administered group: 0.02% benzalkonium administration + 0.3% Sample 1 (total 90 µL) administration

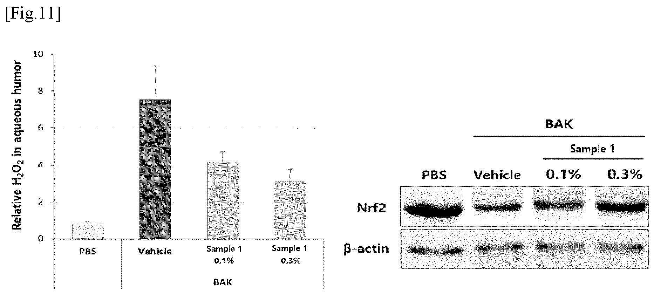

As a result, as shown in FIG. 11, H₂O₂ significantly increased in the control group (Vehicle) compared to the normal group (PBS), whereas H₂O₂ significantly decreased depending on the concentration of the sample in the sample administered groups (0.1% Sample 1 and 0.3% Sample 1) compared to the control group (approximately 40-70% decrease). In addition, while Nrf2 expression significantly decreased in the control group compared to the normal group, Nrf2 expression significantly increased depending on the concentration of the sample in the sample treated group compared to the control group.

### [Example 9] Analysis 2 on safety as an eye drop composition

In order to identify the safety of the lipo-hydroxamic acid derivative prepared above as an eye drop composition, Samples 6, 11, and 16 were set as the first, second, and third administration substances, respectively, and administered to New Zealand White rabbits (Pizhou dongfang Breeding Co., Ltd) six times per day at four-day intervals. Specifically, Sample 6 was administered six times in a single day, followed by a four-day rest period; then Sample 11 was administered in the same manner, followed by another four-day rest period, after which Sample 16 was administered in the same manner (dosage per administration: 50 µL/eye; 100 µL/head (both eyes), daily dosage: 600 µL/head, total dosage per animal: 1.8 mL/head). During the 14-day observation period after the third administration, general symptoms including death were observed, and body weight was measured. Gross necropsy was performed upon completion of the observation period.

As a result, no dead individual was shown during the observation period, with no general symptoms due to the samples. Furthermore, no abnormalities were detected in the gross pathological examination of any of the animals. Notably, even upon administration of Sample 16 at the highest concentration (3 mg/mL), no adverse reactions were observed. From the above results, it was found that the lipo-hydroxamic acid derivatives (Samples 6, 11, and 16) prepared in Experimental Example 1 had a maximum tolerated dose for rabbits exceeding 1.8 mg/head.

The above description of the present disclosure is for illustrative purposes only, and those skilled in the art to which the present disclosure pertains will appreciate that the present disclosure may be easily modified into other specific forms without changing the technical idea or essential features of the present disclosure. Therefore, it should be understood that embodiments described above are exemplary in all respects and not limiting.

The scope of the present disclosure is indicated by the claims set forth below, and all changes or modified forms derived from the meaning and scope of the claims and their equivalent concepts should be interpreted as being included in the scope of the present disclosure.

## Claims

1. A pharmaceutical composition for preventing or treating an ocular disease, comprising a compound comprising a lipo-hydroxamic acid derivative represented by the following Chemical Formula 1 or Chemical Formula 2 or a pharmaceutically acceptable salt, tautomer, stereoisomer, or optical isomer thereof as an active ingredient: 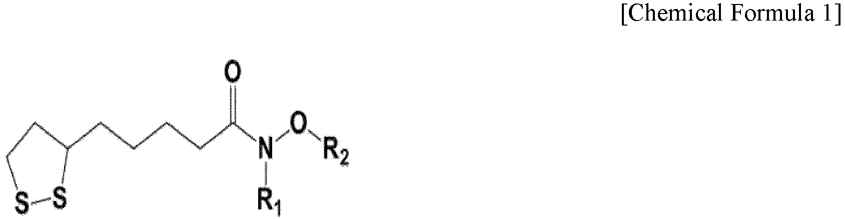 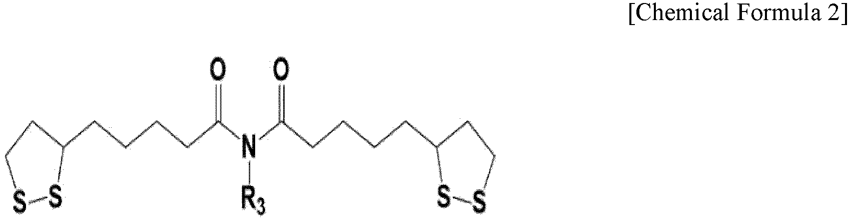 wherein, in the Chemical Formula 1 and Chemical Formula 2, R₁, R₂, and R₃ are the same or different and each independently any one of hydrogen, hydroxy, (C1-C10)alkyl, (C1-C10)alkoxy, and (C1-C10)alkyl substituted with 1 to 5 hydroxy groups.

2. The pharmaceutical composition of claim 1, wherein, in the Chemical Formula 1, R₁ is any one of hydrogen, hydroxy, and (C1-C5)alkyl, and R₂ is any one of hydroxy, (C1-C5)alkyl, and -(C1-C5)alkyl-hydroxy.

3. The pharmaceutical composition of claim 1, wherein, in the Chemical Formula 1, R₁ is hydrogen or methyl, and R₂ is ethyl or -ethyl-hydroxy.

4. The pharmaceutical composition of claim 1, wherein the compound represented by Chemical Formula 1 is selected from the group of following compounds:
1) 5-[1,2]Dithiolan-3-yl-pentanoic acid (2-hydroxy-ethoxy)-amide;
2) 5-[1,2]Dithiolan-3-yl-pentanoic acid ethoxy-amide; and
3) 5-[1,2]Dithiolan-3-yl-pentanoic acid (2-hydroxy-ethoxy)-methyl-amide.

5. The pharmaceutical composition of claim 4, wherein the 5-[1,2]dithiolan-3-yl-pentanoic acid (2-hydroxy-ethoxy)-amide is (R)-5-[1,2]dithiolan-3-yl-pentanoic acid (2-hydroxy-ethoxy)-amide, the 5-[1,2]dithiolan-3-yl-pentanoic acid ethoxy-amide is (R)-5-[1,2]dithiolan-3-yl-pentanoic acid ethoxy-amide, and the 5-[1,2]dithiolan-3-yl-pentanoic acid (2-hydroxy-ethoxy)-methyl-amide is (R)-5-[1,2]dithiolan-3-yl-pentanoic acid (2-hydroxy-ethoxy)-methyl-amide.

6. The pharmaceutical composition of claim 1, wherein, in the Chemical Formula 2, R₃ is any one of hydrogen, hydroxy, and (C1-C5)alkyl.

7. The pharmaceutical composition of claim 1, wherein, in the Chemical Formula 2, R₃ is hydrogen or hydroxy.

8. The pharmaceutical composition of claim 1, wherein the compound represented by Chemical Formula 2 is selected from the group of following compounds:
4) 5-[1,2]Dithiolan-3-yl-pentanoic acid (5-[1,2]dithiolan-3-yl-pentanoyl-amide); and
5) 5-[1,2]Dithiolan-3-yl-pentanoic acid (5-[1,2]dithiolan-3-yl-pentanoyl-hydroxy-amide).

9. The pharmaceutical composition of claim 1, wherein the ocular disease is one or more selected from the group consisting of corneal dystrophy, glaucoma, cataract, keratitis, conjunctivitis, and scleritis.

10. A health functional food composition for preventing or ameliorating an ocular disease, comprising a compound comprising a lipo-hydroxamic acid derivative represented by the following Chemical Formula 1 or Chemical Formula 2 or a pharmaceutically acceptable salt, tautomer, stereoisomer, or optical isomer thereof as an active ingredient: 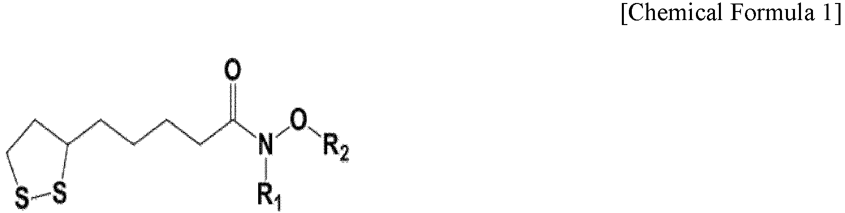 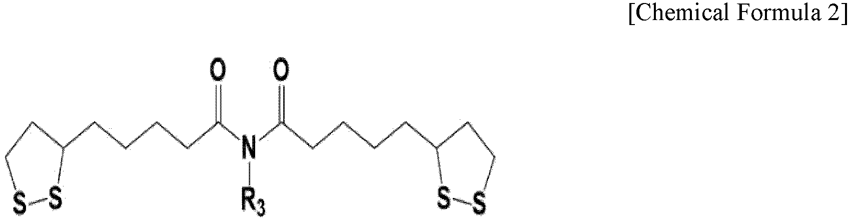 wherein, in the Chemical Formula 1 and Chemical Formula 2, R₁, R₂, and R₃ are the same or different and each independently any one of hydrogen, hydroxy, (C1-C10)alkyl, (C1-C10)alkoxy, and (C1-C10)alkyl substituted with 1 to 5 hydroxy groups.

11. The health functional food composition of claim 10, wherein the ocular disease is one or more selected from the group consisting of corneal dystrophy, glaucoma, cataract, keratitis, conjunctivitis, and scleritis.
